(19) Europäisches Patentamt
European Patent Office
Office européen des brevets

(11) **EP 2 677 927 B1**

(12) **EUROPEAN PATENT SPECIFICATION**

(45) Date of publication and mention
of the grant of the patent:
**24.08.2016 Bulletin 2016/34**

(51) Int Cl.:
***A61B 5/08*** (2006.01)

(21) Application number: **12701265.6**

(86) International application number:
**PCT/GB2012/050134**

(22) Date of filing: **23.01.2012**

(87) International publication number:
**WO 2012/114080 (30.08.2012 Gazette 2012/35)**

(54) **RESPIRATION MONITORING METHOD AND SYSTEM**

ATMUNGSÜBERWACHUNGSVERFAHREN UND -SYSTEM

PROCÉDÉ ET SYSTÈME DE SURVEILLANCE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.02.2011 GB 201103008**

(43) Date of publication of application:
**01.01.2014 Bulletin 2014/01**

(73) Proprietor: **Toumaz UK Limited
Milton Park
Abingdon, Oxfordshire OX14 4SA (GB)**

(72) Inventors:
 • **HERNANDEZ-SILVEIRA, Miguel
 Oxfordshire OX14 4SA (GB)**
 • **ANG, Su-Shin
 Oxfordshire OX14 4SA (GB)**
 • **BURDETT, Alison
 Oxford
 Oxfordshire OX2 0AU (GB)**

(74) Representative: **Lind, Robert
Marks & Clerk LLP
Fletcher House
Heatley Road
The Oxford Science Park
Oxford OX4 4GE (GB)**

(56) References cited:
**US-A1- 2005 148 895    US-A1- 2008 275 349
US-A1- 2009 326 871**

## Description

Field of the Invention

This invention relates to a respiration monitoring method and a system for carrying out the same. Aspects of the invention also relate to a processor for a respiration monitoring system.

Background to the Invention

Many systems for processing respiration signals and extracting patient respiration rates involve techniques that demand high computational power due their mathematical complexity and/or require multiple sensors for the simultaneous acquisition of reference signals used to reduce noise levels in the signals of interest. However, because such systems require long processing times and large memories, they are unsuitable for use in low-cost wearable wireless devices which are battery-operated and therefore require low power consumption.

It is also known to determine respiration rates by employing simple frequency or time-domain analysis based on zero-crossing and/or threshold-based peak detectors. While these techniques can be employed in relatively accurate devices with low computational cost, they rely on a history of continuously acquired correct breath detections for adjusting threshold levels and other variables so as to permit future correct breath detections. In addition, these simple time-domain algorithms are incapable of identifying either those signals corrupted by patient motion or those that are erratic and clinically irrelevant such as those resulting from talking, coughing or swallowing.

An example of such a known system is described in US 2008/275349 which describes an apparatus including at least one sensor, configured to sense a physiological parameter of a subject and to sense large body movement of the subject, an output unit, and a control unit. The control unit of the apparatus is configured to monitor a condition of the subject by analyzing the physiological parameter and the sensed large body movement, and to drive the output unit to generate an alert upon detecting a deterioration of the monitored condition.

The control unit removes segments of the sensed signal which are contaminated with movement or other artifacts, while retaining sufficient data for a statistically significant analysis.

It is therefore an aim of the present invention to provide a respiration monitoring method and system that addresses at least some of the aforementioned problems.

Summary of the Invention

According to a first aspect of the present invention there is provided a method of determining a respiration rate from a signal representative of a recorded respiration episode, the method comprising identifying two or more uncorrupted segments within said signal, the uncorrupted segments being separated by corrupted segments, identifying the longest of the uncorrupted segments or the segment with a lowest average absolute deviation (AAD) between respiration peaks or troughs, and determining a respiration rate from the longest uncorrupted segment or the segment with the lowest AAD.

Embodiments of the present invention therefore provide a method capable of selecting and processing only good quality sections of data from a respiratory waveform. This not only reduces the computational and power requirements for the system but also ensures that the respiration rate is not inadvertently affected by data that may have been corrupted, for example, by motion, talking, coughing or swallowing.

The present invention provides two alternative methods for obtaining a respiration rate from the uncorrupted signals. In both cases, the calculation of the respiration rate is based solely upon uncorrupted segments of data. In the case where the longest uncorrupted segment is used, the method will be less computationally intensive and in the case where the segment with the lowest AAD is used, the calculated respiration rate may be more representative of the actual respiration rate.

It will be understood that in the method of the present invention the respiration rate is calculated as a single value number obtained from an epoch of data.

The method may comprise the step of a user selecting, within that epoch of data, whether the longest uncorrupted segment will be used to calculate the respiration rate or whether the segment with the lowest AAD will be used to calculate the respiration rate.

Where more than one segment is identified as having the same longest length, the earliest identified segment may be chosen as the longest uncorrupted segment.

Where more than one segment is identified as having the same lowest AAD, the earliest identified segment may be chosen as the segment with the lowest AAD.

The uncorrupted segments may be identified dynamically or retrospectively.

The method may further comprise deriving at least one threshold from said signal and/or said uncorrupted segments and using the at least one threshold in any or all of the steps above.

The method may further comprise the step of communicating the respiration rate to a base station. The respiration rate may be wirelessly communicated to the base station.

The step of identifying the uncorrupted segments may comprise checking whether a gradient and/or amplitude of the signal does not exceed a pre-determined threshold.

The step of identifying the uncorrupted segments may comprise identifying peaks and/or troughs in the signal, obtaining intervals between adjacent peaks and/or adjacent troughs, computing changes in said intervals and determining whether such changes exceed a pre-determined threshold.

The step of identifying the uncorrupted segments may comprise calculating a first average absolute deviation between adjacent peaks and a second average absolute deviation between adjacent troughs, combining the first and second average absolute deviations to obtain a normalised threshold and determining whether either of the first or second average absolute deviations exceeds the normalised threshold. The first average absolute deviation may be calculated by identifying peaks in the signal, obtaining intervals between adjacent peaks, calculating a mean peak interval, and computing the average absolute deviation of said intervals. The second average absolute deviation may be calculated by identifying troughs in the signal, obtaining intervals between adjacent troughs, calculating a mean trough interval, and computing the average absolute deviation of said intervals. The step of combining the first and second average absolute deviations may comprise multiplying the sum of the first and second average absolute deviations by a weighting factor.

The method may further comprise pre-processing the uncorrupted segments prior to determining the respiration rate.

The step of pre-processing may comprise one or more of:

> (i) data centring;
> (ii) gain adjustment;
> (iii) passing the segments through a self-tunable filter;
> (iv) passing the segments through a low and/or high pass filter.

The self-tunable filter may be configured to cancel noise produced by dynamic changes in the impedance of a monitored heart as it expands and contracts. The self-tunable filter may use heart rate information to adjust a cut-off frequency in accordance with a frequency of concurrent cardiogenic contaminants.

The method may further comprise the step of respiration event checking comprising one or more of:

> (i) identifying peaks and/or troughs outside a noise margin window;
> (ii) classifying a peak as an inhalation event only if it is preceded by an exhalation trough;
> (iii) classifying a trough as an exhalation event only if it is preceded by an inhalation peak;
> (iv) discarding an inhalation and/or exhalation event if it would result in a respiration rate exceeding an expected maximum;
> (v) discarding an inhalation and/or exhalation event if its amplitude is less than any one of 20%, 30%, 40% or 50% of the previous inhalation/exhalation event.

Advantageously, the method may comprise determining whether an inhalation and/or exhalation event has an amplitude which is at least a preset percentage of the amplitude of the previous inhalation/exhalation event. The method may therefore not comprise use of any average amplitude values.

The method may further comprise performing a periodicity check, after the step of respiration event checking, to determine whether the variability in intervals between two adjacent peaks and/or two adjacent troughs exceeds a pre-determined threshold.

The method may further comprise the step of validity checking comprising calculating the number of valid peaks and/or troughs detected and evaluating whether the number is greater than or equal to a pre-determined minimum required to calculate a valid respiration rate.

The method may further comprise verifying whether an average absolute deviation for the interval between adjacent valid peaks does not exceed a preset value and/or an average absolute deviation for the interval between adjacent valid troughs does not exceed a preset value.

The respiration rate may be calculated as the mean or median respiratory rate obtained using valid inhalation and/or exhalation events obtained from said identified uncorrupted segment.

The method may further comprise generating an error signal when the signal representing the respiration episode does not contain at least two segments of contiguous uncorrupted data and/or when any calculated values exceed their respective thresholds.

According to a second aspect of the present invention there is provided a respiration monitoring system for monitoring respiration to determine a respiration rate from a signal representative of a recorded respiration episode, the system comprising:

> a sensor for generating a signal representative of a recorded respiration episode; and
> a processor configured for:
>
>> identifying two or more uncorrupted segments within said signal, the uncorrupted segments being separated by corrupted segments,
>> identifying the longest of the uncorrupted segments or the segment with a lowest average absolute deviation (AAD) between respiration peaks or troughs, and
>> determining a respiration rate from the longest uncorrupted segment or the segment with the lowest AAD.

The sensor may comprise an impedance pneumography device configured to generate said signal.

The system may be configured as a wearable wireless device.

The system may be configured a low-power battery-operated disposable device.

According to a third aspect of the present invention there is provided a processor for a respiration monitoring system for monitoring respiration to determine a respiration rate from a signal representative of a recorded respiration episode, said processor configured for:

> identifying two or more uncorrupted segments within said signal, the uncorrupted segments being separated by corrupted segments,
> identifying the longest of the uncorrupted segments or the segment with a lowest average absolute deviation (AAD) between respiration peaks or troughs, and
> determining a respiration rate from the longest uncorrupted segment or the segment with the lowest AAD.

The features described above in relation to one aspect of the invention may equally be applied to other aspects of the invention and vice versa.

Brief Description of the Drawings

The various aspects of the invention will now be described in relation to the accompanying drawings in which:

> Figure 1 shows flow diagram for a method of monitoring respiration to determine a respiration rate according to an embodiment of the present invention;
> Figure 2 shows a block diagram of a respiration monitoring system for carrying out the method illustrated in Figure 1 in accordance with an embodiment of the present invention;
> Figure 3 illustrates a more specific embodiment of the general method shown in Figure 1, in accordance with another embodiment of the present invention; and
> Figure 4 illustrates a signal representing a respiration episode and how this is analysed in accordance with embodiments of the invention.

Detailed Description of Certain Embodiments

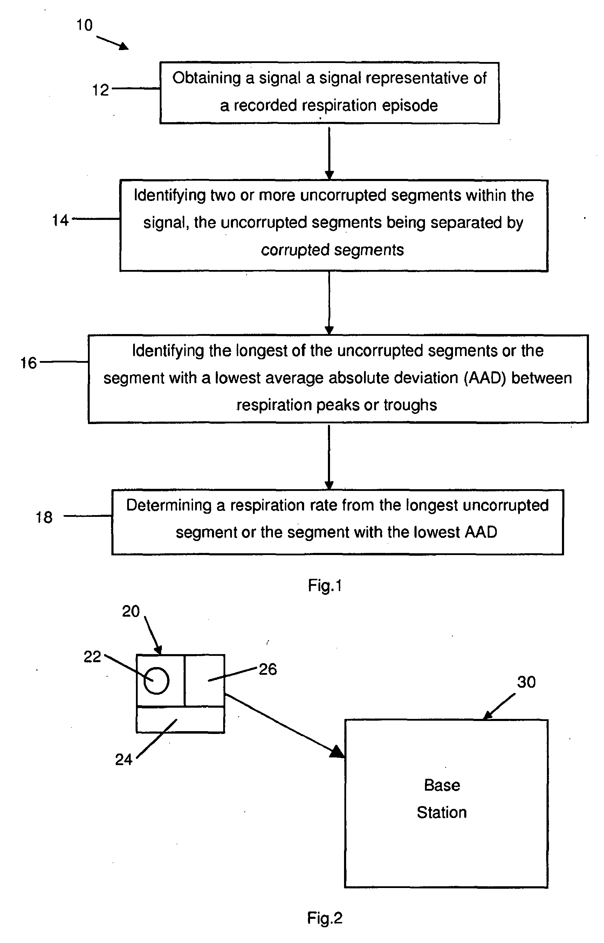

Figure 1 shows a flow diagram for a method 10 of determining a respiration rate from a signal representative of a recorded respiration episode, according to an embodiment of the present invention.

The method 10 comprises a first step 12 of obtaining a signal representative of a respiration episode. A second step 14 of identifying two or more uncorrupted segments within the signal, the uncorrupted segments being separated by corrupted segments, follows. A third step 16 is then undertaken which comprises identifying the longest of the uncorrupted segments or the segment with a lowest average absolute deviation (AAD) between respiration peaks or troughs before a fourth step 18 of determining a respiration rate from the longest uncorrupted segment or the segment with the lowest AAD is carried out.

Accordingly, the method 10 can be used to determine whether a signals representing a respiration episode (e.g. of 10 minutes duration) comprises two or more segments of continuous uncorrupted data, which can be extracted, conditioned (e.g. pre-processed) and compared with other similarly obtained data segments to calculate a respiration rate from the longest or most consistent (i.e. cleanest) segment. This embodiment therefore provides a method 10 which is capable of selecting and processing only good quality sections of data from a respiratory waveform. This has advantages in reducing the computational and power requirements for a system carrying out the method, as well as ensuring that the respiration rate is more accurately calculated since corrupted data (e.g. affected by motion, talking, coughing or swallowing) is reduced or eliminated prior to the calculation of the respiration rate. In addition, there is no requirement to wait until a complete uncorrupted episode of a specific length (e.g. 1 min) has been obtained before a respiration rate can be calculated because usable parts of corrupted episodes can be extracted to provide a respiration rate within a shorter period of time.

Figure 2 illustrates a respiration monitoring system 20 for carrying out the method 10 of Figure 1 in accordance with an embodiment of the present invention. The system 20 comprises a sensor 22 for generating a signal representing a respiration episode and a processor 24. The processor 24 is configured for identifying at least two uncorrupted segments within the signal, the uncorrupted segments being separated by corrupted segments. The processor 24 is also configured for identifying the longest of the uncorrupted segments or the segment with a lowest average absolute deviation (AAD) between respiration peaks or troughs before determining a respiration rate from the longest uncorrupted segment or the segment with the lowest AAD.

In the embodiment shown in Figure 2, the system 20 is configured as a low-power battery-operated wearable device having a transceiver 26 configured for wireless communication with a base station 30 (which may be constituted by a remote computer). In other embodiments, other communication methods may be employed such as those operated over fixed lines, for example, in local area networks.

The sensor 22 comprises a single-lead electrode impedance pneumography device configured to generate the signal to be processed by the processor 24. The respiration rate calculated by the processor 24 is then transmitted via the transceiver 26 to the base station 30. Local processing of the respiratory data (by the sensor 22) allows less data to be transmitted to the base station 30, extending the battery life of the sensor 22. It will be understood that a plurality of systems 20 may be configured to communicate with the base station 30 so that, for example, the base station 30 can provide respiration rates for a number of patients wearing the systems 20 on their chests.

The system 20 may be configured to record the patient's respiratory activity in a discontinuous but cyclical fashion. For example, the system may record a full respiration episode of 1 minute duration and begin processing this to determine if it contains any segments of continuous uncorrupted data for at least 10 seconds. In other embodiments the minimum period of uncorrupted data required to calculate a valid respiration rate from the segment may be 1 minute. If the processor does not obtain at least the minimum period (in this case, 10 seconds) of continuous uncorrupted data from a single respiration episode the sensor 22 will record another respiration episode and the above process will be repeated until at least the minimum period of uncorrupted data is obtained. Once an uncorrupted segment has been so obtained, it will be further processed before being used to calculate the respiration rate.

The processor 24 may be configured to periodically transmit to the base station 30 a result for the respiration rate. If a suitable period of uncorrupted data has been obtained the result will be a valid respiration rate in breaths per minute (BrPM). In all other circumstances, the result may take the form of a message code indicating that the signal was erratic or invalid and so no valid respiration rate could be obtained.

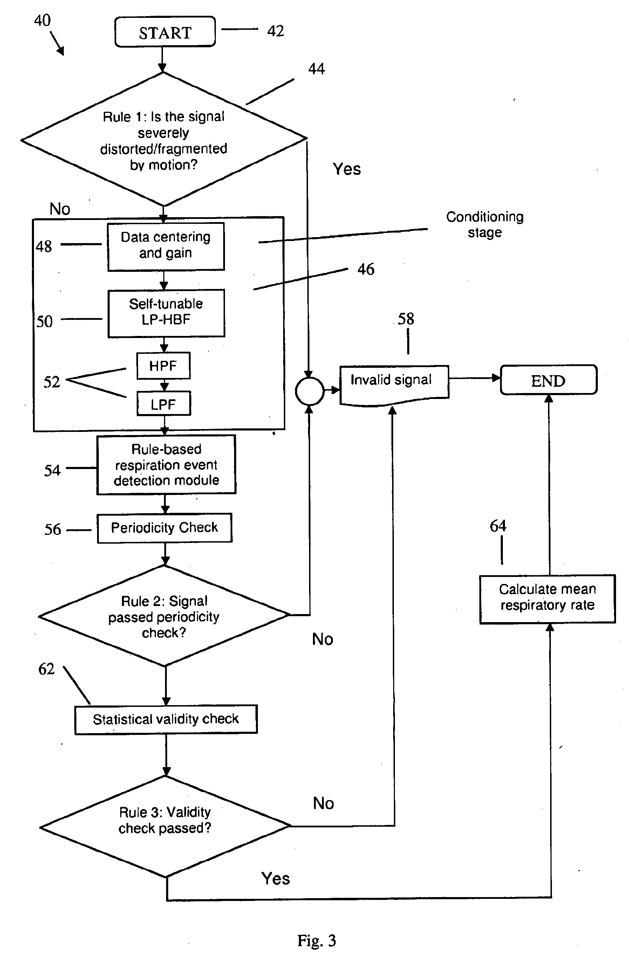

Figure 3 illustrates a more specific embodiment of the general method shown in Figure 1, in accordance with a particular embodiment of the present invention. The method 40 shown in Figure 3 includes a number of rules which are checked at different stages of the process in order to reject data segments either severely corrupted by motion or not clinically relevant. The method 40 is started 42 as soon as a signal for a 1 minute respiration episode has been obtained from the sensor 22 and stored in a buffer (not shown). This data is first passed through a step 44 of determining whether the signal comprises at least one segment of continuous uncorrupted data for at least 10 seconds (as specified in a stored value representing the pre-determined minimum segment duration). In other embodiments, the minimum segment duration may be set to 5, 15, 20 or 30 seconds. Similarly, the respiration episode need not be limited to 1 minute and may, for example, last 30 seconds, 2 minutes, 3 minutes or 5 minutes. Furthermore, the respiration episode may or may not have a duration equivalent to the minimum period of uncorrupted data.

In some embodiments, step 44 may comprise checking whether the signal is severely distorted and/or fragmented by motion. Accordingly, step 44 may be carried out by a motion detector and/or discriminator module. As well as being able to distinguish between entirely severely corrupted and clean respiration signals, this module is also capable of partially recovering uncorrupted segments of genuine respiration data. Thus, the module evaluates whether the respiration episode contains at least one 10 second segment of continuous (or contiguous) uncorrupted data. This step 44 can be carried out by analysing the slope and amplitude characteristics of the waveform, rejecting entire corrupted episodes and accepting segments of data from which a reliable respiration rate could potentially be extracted. The accepted episodes and segments of usable data are then stored in a memory (e.g. in an array) for further processing and verification as will be explained in more detail below.

More specifically, the motion detector and discriminator module according to the present embodiment of the invention operates as follows in order to carry out step 44. Firstly, a start pointer and an end pointer are used to initially point to a start position and an end position of a data buffer, which is initially loaded with signal data representing the whole of a respiration episode. The motion (artefact) detector module then carries out the following stages in order to update the start and end pointers so that they encompass only uncorrupted segments of the data:

    1) Gradient and amplitude check;
    2) Analysis of step changes in periodicity;
    3) Calculation of Average Absolute Deviation (AAD) in the periodicity of the waveform.

Stage 1 - Gradient and amplitude check

The gradient of the signal is checked to ensure that it does not exceed a pre-determined gradient threshold. In this embodiment, the gradient threshold is stored in memory and is set to correspond to that of a perfect sinusoidal wave having a frequency of 2Hz. If any portions of the signal have a gradient that is steeper than the gradient threshold, such portions are rejected and the start and end pointers are updated to encompass only data that does not exceed the gradient threshold.

Similarly, the amplitude of the signal is checked to ensure that it does not exceed a pre-determined amplitude threshold. In this embodiment, the amplitude threshold is stored in memory and is set to correspond to two thirds of a maximum possible amplitude value. If any portions of the signal have an amplitude that exceeds the amplitude threshold, such portions are rejected and the start and end pointers are updated to encompass only data that does not exceed the amplitude threshold.

Stage 2 - Analysis of step changes in periodicity

A peak detector (as described in the prior art) is next run through the segment of data identified in the previous stage rather than the original 60 seconds worth of data in order to save processing time and battery life. The output from the peak detector is a set of peak and trough locations. Changes in the peak to peak (and/or trough to trough) intervals are then computed and are deemed to be motion artefacts if they exceed a threshold S calculated in accordance with equation 1 below, in which $T_i$ represents a peak to peak (or trough to trough) interval, subscript i represents a particular interval in time, and $\max(T_i, T_{i-1})$ represents the maximum difference between adjacent peak to peak (or trough to trough) intervals.

$$\frac{|T_i - T_{i-1}|}{\max(T_i, T_{i-1})} \leq S \qquad (1)$$

The start and end pointers are then updated to exclude data not falling within the threshold limit.

If, during either of the above two stages, the start and end pointers are adjusted to encompass less than 10 seconds of data, the module will generate a signal to indicate that a valid respiration rate cannot be calculated and the sensor 22 may be instructed to obtain a signal for a further 1 minute respiration episode.

Stage 3 - Calculation of Average Absolute Deviation (AAD)

If, however, more than 10 seconds of data is encompassed by the start and end pointers at the end of the above two stages, the Average Absolute Deviation (AAD) of the peak to peak (arid trough to trough) intervals will be computed using equation (2), where N is the total number of peak to peak (or trough to trough) intervals in the data set and $\mu$ is the mean interval value.

$$AAD = (1/N) \sum_{i=0}^{N-1} |T_i - \mu| \qquad (2)$$

A normalized threshold value L is then determined in accordance with equation (3) by multiplying the sum of the AAD values for the peaks and the troughs by a weighting factor of 0.15. (In other embodiments, a different weighting fact may be used.)

$$L = 0.15(AAD_{peaks} + AAD_{troughs}) \qquad (3)$$

An invalid signal is generated if either of the $AAD_{peaks}$ or $AAD_{troughs}$ values exceeds the normalised threshold value L calculated in equation (3).

In addition, if during this stage, the start and end pointers are adjusted to encompass less than 10 seconds of data, the module will generate a signal to indicate that a valid respiration rate cannot be calculated and the sensor 22 may be instructed to obtain a signal for a further 1 minute respiration episode. However, if after all three stages described above, the start and end pointers still encompass at least 10 seconds of uncorrupted data, this segment is further processed as will be described below in order to calculate a respiratory rate.

The next step of the method 40 is to perform a conditioning stage 46 on the data encompassed between the start and end pointers. Note, depending on the levels of corruption in the original respiration signal, this process may be applied to the whole of the episode (which has passed through the above stages) or only on a segment of the original signal which has survived the above stages.

The conditioning stage 46 comprises data centring (to remove any offset associated with the signal) and gain adjustment 48. It is noted that data centring is important not only because subsequent processes (which will be described below) require positive and negative values but also because it provides a solution for dealing with input offset errors which can vary across devices.

The conditioning stage 46 also comprises passing the signal through a self-tunable digital notch filter 50 to cancel in-band noise produced by dynamic changes in the impedance of the heart as it contracts and expands. Such a filter can be described as a 'heat-bump' filter which uses heart rate information to adjust its cut-off frequency in accordance with the frequency of concurrent cardiogenic contaminants. Analogue hardware filters of this type are known and have been adapted as software digital filters for use in embodiments of the present invention.

Once heart artifacts have been removed, the signal is further processed by first-order recursive low and high pass filters 52, whose coefficients are calculated for a sampling rate of 25Hz and cut-off frequencies of 2Hz and 0.1 Hz respectively. Thus, other sources of contamination outside of the respiration bandwidth (e.g. from muscle and mains noise) are successfully removed, allowing the method to accurately detect respiration activity within 4 and 120 BrPM.

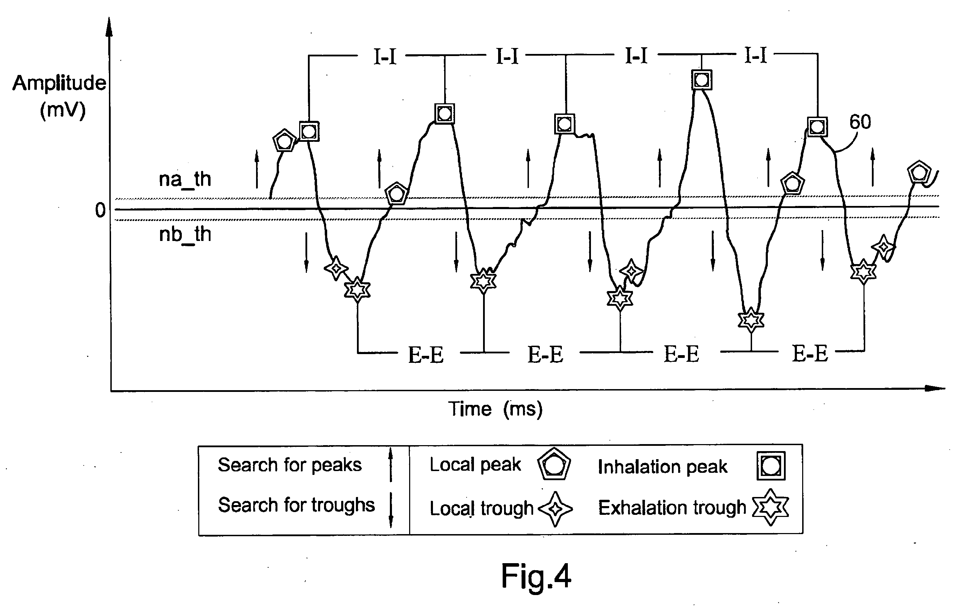

The next step is to pass the signal through the respiration events detection module 54 once more. In embodiments of the invention, this module 54 comprises a three-point sliding window peak/trough detector and a routine that identifies respiration events (i.e. inhalation peaks and exhalation troughs) based on a number of rules and static thresholds associated with typical amplitude and time domain features of impedance pneumography respiration signals 60, as illustrated in Figure 4. More specifically, the module searches for peaks and troughs outside of a noise margin window (delimited by hysteresis borders na_th and nb_th in Figure 4). As illustrated, inhalation to inhalation (I-I) and exhalation to exhalation (E-E) intervals are calculated as the distances between adjacent maximum peak values and adjacent minimum trough values, respectively. These intervals are stored in buffers and used later for the calculation of the respiration rate from either the mean or median values of the I-I and E-E intervals.

In addition to the artefact and other noise checks described above, this module considers further possible existence of undetected and unfiltered noise. Accordingly, the module 54 implements a,number of conditions or rules to avoid misdetection and false detection of respiration events. These rules are listed below (in no particular order):

1. A peak is classified as an inhalation event only if it is preceded by an exhalation trough.

2. A trough is classified as an exhalation event only if it is preceded by an inhalation peak.

3. An inhalation or exhalation event is regarded as genuine and stored in its respective buffer if it has not occurred quicker than a time limit threshold corresponding to that which would occur for a maximum expected respiration rate (e.g. 120 BrPM).

4. An inhalation event or exhalation event is regarded as valid if its amplitude is at least 20% of the previous one and it is outside of the preset noise hysteresis borders (*n4_th* and *nb_th*). Some existing methods employ average amplitude values calculated over significantly longer time periods (i.e. including many inhalation and exhalation events) in order to classify respiration events. Such techniques are often therefore unable to handle sudden mean level changes in amplitude (as can be experienced when analysing relatively small isolated segments of data in accordance with the present invention) and so missed breath detections can occur with these standard methods. On the contrary, the condition employed here permits rapid adaptation of the system to changes in amplitude which can occur particularly when using discontinuous segments of data from which successive respiration rates can be calculated.

In relation to the above, it is clear from Figure 4 that some local peaks and local troughs may be identified during the analysis of the signal but as these peaks and troughs do not meet all of the requirements listed above, they will not be classified as inhalation peaks or exhalation troughs and will therefore be discounted from any calculation of a respiration rate.

After the respiration events detection module stage 54, the signal undergoes a periodicity check 56 where remainder noise is searched for, which is only identifiable by its non-periodic behaviour. During this stage 56, the system makes use of the inhalation and exhalation events identified by the respiration events detection module 54. During the periodicity check 56, the system searches for periodicity irregularities in inhalation to inhalation (I-I) and exhalation to exhalation (E-E) intervals. The periodicity module essentially looks for substantial variability between signal peaks (or troughs). More specifically, the periodicity module checks whether the absolute difference between every two adjacent signal peak to peak (or trough to trough) intervals (normalised by the position of the furthest one) exceeds a preset threshold, in accordance with equation (1) above.

If the signal (or segment) does not pass the periodicity check 56, an invalid signal 58 is generated and the sensor 22 may be instructed to obtain a signal for a further 1 minute respiration episode for processing as described above. If, however, the signal (or segment) passes the periodicity check 56 it proceeds to the next stage. The uncorrupted segments of data are therefore first identified by step 44, before conditioning in step 46, and are once again analysed during the periodicity check 56 to ensure that the data is still in a suitable condition for calculating a respiration rate. The size of each segment is reduced accordingly at each stage in order to filter out unwanted or erroneous parts of the signal.

The signal is then passed through a statistical validity check 62 to determine whether enough respiration peaks and troughs have been detected (i.e. whether the segment is still of a sufficiently long length) in order to calculate a breath rate per minute (BrPM). In addition, this step 62 may verify whether the average absolute deviation (AAD) between neighbouring inhalation events and/or neighbouring exhalation events is reasonable (i.e. does not exceed a preset threshold). The use of AADs in this step is advantageous because it can provide an indication that is close to a standard Gaussian deviation (e.g. within about 80% of standard deviation) but with significant computational efficiency. This is because commonly used statistical measures such as variance or standard deviation involve compute and energy intensive arithmetic (e.g. power and square root) calculations whereas the AAD can be implemented using only sums and multiplications. In addition, the AAD is not as sensitive to outliers and is more efficient as an estimate of a population parameter in real-life situations where the data is not distributed normally. Accordingly, it is believed that the use of AAD values is important in the assessment of the signal integrity. It is also advantageous that AAD calculations can be easily implemented in fixed-point microcontroller processors as employed in systems like that shown in Figure 2.

If the signal does not pass the statistical validity check 62, an invalid signal 58 is generated and the sensor 22 may be instructed to obtain a signal for a further 1 minute respiration episode for processing as described above.

If the signal passes the statistical validity check 62, the system will then calculate the mean (or median) respiratory rate in step 64. This is performed using the stored I-I and E-E intervals obtained from either the longest uncorrupted segment or the segment with the lowest AAD, which is stored in the buffer after completion of the above steps. The respiration rate may then be transmitted to a base station as described above.

Advantages of the present invention are that computational savings, cost savings, power savings and time savings may be realised since the method is largely only required to analyse uncorrupted segments of data of a useful size. At any point, if at least a minimum duration (e.g. 10 seconds) of uncorrupted data is not available, the algorithm will terminate and the sensor 22 may be instructed to obtain a further set of data for processing.

It should be noted that all of the thresholds and limits employed in the embodiments described above were determined from simulations and from experimental evaluation of a statistically acceptable number of respiration episodes obtained from healthy individuals and patients. In other embodiments, other threshold levels may be set as appropriate.

It will also be appreciated by persons skilled in the art that various modifications may be made to the above embodiments without departing from the scope of the present invention. For example, features described in relation to one embodiment may be incorporated into another embodiment or vice versa.

**Claims**

1. A method of determining a respiration rate from a signal representative of a recorded respiration episode, the method comprising providing a starter pointer and an end pointer to point to a start position and an end position of the signal representative of the episode, and **characterised by** updating the start and end pointers to identify the start and end positions of two or more uncorrupted segments within said signal, the uncorrupted segments being separated by corrupted segments, identifying the longest of the uncorrupted segments or the segment with a lowest average absolute deviation, AAD, between respiration peaks or troughs, and determining a respiration rate from the longest uncorrupted segment or the segment with the lowest AAD.

2. The method according to claim 1 further comprising the step of a user selecting whether the longest uncorrupted segment will be used to calculate the respiration rate or whether the segment with the lowest AAD will be used to calculate the respiration rate

3. The method according to claim 1 wherein the uncorrupted segments are identified dynamically or retrospectively.

4. The method according to any preceding claim wherein the step of identifying the uncorrupted segments comprises checking whether a gradient and/or amplitude of the signal does not exceed a pre-determined threshold or the steps of: identifying the uncorrupted segments comprises identifying peaks and/or troughs in the signal, obtaining intervals between adjacent peaks and/or adjacent troughs, computing changes in said intervals and determining whether such changes exceed a pre-determined threshold.

5. The method according to any preceding claim wherein the step of identifying the uncorrupted segments comprises calculating a first average absolute deviation between adjacent peaks and a second average absolute deviation between adjacent troughs, combining the first and second average absolute deviations to obtain a normalised threshold and determining whether either of the first or second average absolute deviations exceeds the normalized threshold.

6. The method according to claim 5 wherein the step of combining the first and second average absolute deviations comprises multiplying the sum of the first and second average absolute deviations by a weighting factor.

7. The method according to any preceding claim further comprising pre-processing the uncorrupted segments prior to determining the respiration rate.

8. The method according to claim 7 wherein the pre-processing comprises one or more of:

    (v) data centring;
    (vi) gain adjustment;
    (vii) passing the segments through a self-tunable filter;
    (viii) passing the segments through a low and/or high pass filter.

9. The method according to any preceding claim comprising the step of respiration event checking comprising one or more of:

    (i) identifying peaks and/or troughs outside a noise margin window;
    (ii) classifying a peak as an inhalation event only if it is preceded by an exhalation trough;
    (iii) classifying a trough as an exhalation event only if it is preceded by an inhalation peak;
    (iv) discarding an inhalation and/or exhalation event if it would result in a respiration rate exceeding an expected maximum;
    (v) discarding an inhalation and/or exhalation event if its amplitude is less than any one of 20%, 30%, 40% or 50% of the previous inhalation/exhalation event.

10. The method according to claim 9 further comprising performing a periodicity check to determine whether the variability in intervals between two adjacent peaks and/or two adjacent troughs exceeds a pre-determined threshold.

11. The method according to any preceding claim further comprising the step of validity checking comprising calculating a number of valid peaks and/or troughs detected and evaluating whether the number is greater than or equal to a pre-determined minimum required to calculate the valid respiration rate.

12. The method according to any preceding claim further comprising verifying whether an average absolute deviation for an interval between adjacent valid peaks does not exceed a preset threshold and/or an average Absolute deviation for an interval between adjacent valid troughs does not exceed a preset threshold.

13. The method according to any preceding claim wherein the respiration rate is calculated as the mean or median respiratory rate obtained using inhalation and/or exhalation events obtained from said identified uncorrupted segment.

14. The method according to any preceding claim further comprising generating an error signal when the signal representing the respiration episode does not contain at least two segments of contiguous uncorrupted data and/or when any calculated values exceed their respective thresholds.

15. A respiration monitoring system (20) for monitoring respiration to determine a respiration rate from a signal representative of a recorded respiration episode, the system (20) comprising:

a sensor (22) for generating a signal representative of a recorded respiration episode; and
a processor (24) configured for:

providing a starter pointer and an end pointer to point to a start position and an end position of the signal representative of a recorded respiration episode;

the system (20) being **characterised by** the processor (20) being configured for:

updating the start and end pointers to identify the start and end positions of two or more uncorrupted segments within said signal, the uncorrupted segments being separated by corrupted segments,
identifying the longest of the uncorrupted segments or the segment with a lowest average absolute deviation (AAD) between respiration peaks or troughs, and
determining a respiration rate from the longest uncorrupted segment or the segment with the lowest AAD.

16. The system according to claim 15 wherein the sensor (22) comprises an impedance pneumography device configured to generate said signal.

17. The system according to claim 15 or claim 16 configured as a wearable wireless device and/or a low-power battery-operated disposable device.

18. A processor (24) for a respiration monitoring system for monitoring respiration to determine a respiration rate from a signal representative of a recorded respiration episode, said processor (24) configured for:

providing a starter pointer and an end pointer to point to a start position and an end position of the signal representative of a recorded respiration episode, and **characterised by**:

updating the start and end pointers to identify the start and end positions of two or more uncorrupted segments within said signal, the uncorrupted segments being separated by corrupted segments,
identifying the longest of the uncorrupted segments or the segment with a lowest average absolute deviation (AAD) between respiration peaks or troughs, and
determining a respiration rate from the longest uncorrupted segment or the segment with the lowest AAD.

**Patentansprüche**

1. Verfahren zum Bestimmen einer Atemfrequenz aus einem Signal, das für eine aufgezeichnete Atmungsepisode repräsentativ ist, wobei das Verfahren das Bereitstellen eines Startanzeigers und eines Endanzeigers, um auf eine Startposition und eine Endposition des für die Episode repräsentativen Signals zu zeigen, umfasst und **gekennzeichnet ist durch** das Aktualisieren des Start- und des Endanzeigers, um die Start- und die Endpositionen von zwei oder mehr unbeschädigten Segmenten innerhalb des Signals zu identifizieren, wobei die unbeschädigten Segmente **durch** beschädigte Segmente getrennt sind, das Identifizieren des längsten der unbeschädigten Segmente oder des Segments mit einer niedrigsten durchschnittlichen absoluten Abweichung, AAD, zwischen Atmungsspitzen oder -tälern, und das Bestimmen einer Atemfrequenz aus dem längsten unbeschädigten Segment oder dem Segment mit der niedrigsten AAD.

2. Verfahren nach Anspruch 1, das ferner den Schritt umfasst, dass ein Benutzer wählt, ob das längste unbeschädigte Segment verwendet werden wird, um die Atemfrequenz zu berechnen, oder ob das Segment mit der niedrigsten AAD verwendet werden wird, um die Atemfrequenz zu berechnen.

3. Verfahren nach Anspruch 1, wobei die unbeschädigten Segmente dynamisch oder retrospektiv identifiziert werden.

4. Verfahren nach einem der vorhergehenden Ansprüche, wobei der Schritt des Identifizierens der unbeschädigten Segmente das Prüfen, ob eine Steigung und/oder eine Amplitude des Signals nicht einen vorbestimmten Schwellenwert überschreitet, oder die folgenden Schritte umfasst: das Identifizieren der unbeschädigten Segmente umfasst das Identifizieren von Spitzen und/oder Tälern in dem Signal, das Erfassen von Intervallen zwischen benachbarten Spitzen und/oder benachbarten Tälern, das Berechnen von Änderungen in den Intervallen und das Bestimmen, ob solche Änderungen einen vorbestimmten Schwellenwert überschreiten.

5. Verfahren nach einem der vorhergehenden Ansprüche, wobei der Schritt des Identifizierens der unbeschädigten Segmente das Berechnen einer ersten durchschnittlichen absoluten Abweichung zwischen benachbarten Spitzen und einer zweiten durchschnittlichen absoluten Abweichung zwischen benachbarten Tälern, das Kombinieren der ersten und der zweiten durchschnittlichen absoluten Abweichung, um einen normalisierten Schwellenwert zu erhalten, und das Bestimmen, ob eine von der ersten und der zweiten durchschnittlichen absoluten Abweichung den normalisierten Schwellenwert überschreitet, umfasst.

6. Verfahren nach Anspruch 5, wobei der Schritt des Kombinierens der ersten und der zweiten durchschnittlichen absoluten Abweichung das Multiplizieren der Summe der ersten und der zweiten durchschnittlichen absoluten Abweichung mit einem Wichtungsfaktor umfasst.

7. Verfahren nach einem der vorhergehenden Ansprüche, das ferner das Vorverarbeiten der unbeschädigten Segmente vor dem Bestimmen der Atemfrequenz umfasst.

8. Verfahren nach Anspruch 7, wobei das Vorverarbeiten eines oder mehrere von Folgendem umfasst:

   (v) das Datenzentrieren,
   (vi) das Verstärkungseinstellen,
   (vii) das Führen der Segmente durch ein selbstabstimmbares Filter,
   (viii) das Führen der Segmente durch ein Tief- und/oder Hochpassfilter.

9. Verfahren nach einem der vorhergehenden Ansprüche, das den Schritt des Atmungsereignisprüfens umfasst, der eines oder mehrere von Folgendem umfasst:

   (i) das Identifizieren von Spitzen und/oder Tälern außerhalb eines Störabstandsfensters,
   (ii) das Klassifizieren einer Spitze als ein Einatmungsereignis nur, wenn ihm ein Ausatmungstal vorangeht,
   (iii) das Klassifizieren eines Tals als ein Ausatmungsereignis nur, wenn ihm eine Einatmungsspitze vorangeht,
   (iv) das Verwerfen eines Einatmungs- und/oder Ausatmungsereignisses, falls es zu einer Atemfrequenz führen würde, die ein erwartetes Maximum überschreitet,
   (v) das Verwerfen eines Einatmungs- und/oder Ausatmungsereignisses, falls seine Amplitude geringer ist als ein beliebiges von 20 %, 30 %, 40 % oder 50 % des vorherigen Einatmungs-/Ausatmungsereignisses.

10. Verfahren nach Anspruch 9, das ferner das Durchführen einer Periodizitätsprüfung umfasst, um festzustellen, ob die Variabilität in Intervallen zwischen zwei benachbarten Spitzen und/oder zwei benachbarten Tälern einen vorbestimmten Schwellenwert überschreitet.

11. Verfahren nach einem der vorhergehenden Ansprüche, das ferner den Schritt des Gültigkeitsprüfens umfasst, der das Berechnen einer Anzahl von erfassten gültigen Spitzen und/oder Tälern und das Abschätzen, ob die Anzahl größer ist als ein vorbestimmtes, zum Berechnen der gültigen Atemfrequenz erforderliches Minimum oder gleich demselben ist, umfasst.

12. Verfahren nach einem der vorhergehenden Ansprüche, das ferner das Überprüfen, ob eine durchschnittliche absolute Abweichung für ein Intervall zwischen benachbarten gültigen Spitzen nicht einen voreingestellten Schwellenwert überschreitet und/oder eine durchschnittliche absolute Abweichung für ein Intervall zwischen benachbarten gültigen Tälern nicht einen voreingestellten Schwellenwert überschreitet, umfasst.

13. Verfahren nach einem der vorhergehenden Ansprüche, wobei die Atemfrequenz als die Atemmittel- oder -medianfrequenz berechnet wird, erlangt unter Verwendung von Einatmungs- und/oder Ausatmungsereignissen, erlangt aus dem identifizierten unbeschädigten Segment.

14. Verfahren nach einem der vorhergehenden Ansprüche, das ferner das Erzeugen eines Fehlersignals umfasst, wenn das Signal, das die Atmungsepisode repräsentiert, nicht wenigstens zwei Segmente von zusammenhängenden unbeschädigten Daten enthält und/oder wenn beliebige berechnete Werte ihre jeweiligen Schwellenwerte überschreiten.

**15.** Atmungsüberwachungssystem (20) zum Überwachen der Atmung, um eine Atemfrequenz aus einem Signal, das für eine aufgezeichnete Atmungsepisode repräsentativ ist, zu bestimmen, wobei das System (20) Folgendes umfasst:

einen Sensor (22) zum Erzeugen eines Signals, das für eine aufgezeichnete Atmungsepisode repräsentativ ist,
einen Prozessor (24), der für Folgendes konfiguriert ist:

das Bereitstellen eines Startanzeigers und eines Endanzeigers, um auf eine Startposition und eine Endposition des für die Episode repräsentativen Signals zu zeigen, wobei das System (20) **dadurch gekennzeichnet ist, dass** der Prozessor (24) für Folgendes konfiguriert ist:

das Aktualisieren des Start- und des Endanzeigers, um die Start- und die Endpositionen von zwei oder mehr unbeschädigten Segmenten innerhalb des Signals zu identifizieren, wobei die unbeschädigten Segmente durch beschädigte Segmente getrennt sind,
das Identifizieren des längsten der unbeschädigten Segmente oder des Segments mit einer niedrigsten durchschnittlichen absoluten Abweichung (AAD) zwischen Atmungsspitzen oder -tälern und
das Bestimmen einer Atemfrequenz aus dem längsten unbeschädigten Segment oder dem Segment mit der niedrigsten AAD.

**16.** System nach Anspruch 15, wobei der Sensor (22) eine Impedanzpneumographie-Einrichtung umfasst, die dafür konfiguriert ist, das Signal zu erzeugen.

**17.** System nach Anspruch 15 oder Anspruch 16, das als eine tragbare drahtlose Einrichtung und/oder eine batteriebetriebene Einwegeinrichtung niedriger Leistung konfiguriert ist.

**18.** Prozessor (24) für ein Atmungsüberwachungssystem (20) zum Überwachen der Atmung, um eine Atemfrequenz aus einem Signal, das für eine aufgezeichnete Atmungsepisode repräsentativ ist, zu bestimmen, wobei der Prozessor (24) für Folgendes konfiguriert ist:

das Bereitstellen eines Startanzeigers und eines Endanzeigers, um auf eine Startposition und eine Endposition des für die Episode repräsentativen Signals zu zeigen, und **gekennzeichnet durch**:

das Aktualisieren des Start- und des Endanzeigers, um die Start- und die Endpositionen von zwei oder mehr unbeschädigten Segmenten innerhalb des Signals zu identifizieren, wobei die unbeschädigten Segmente **durch** beschädigte Segmente getrennt sind,
das Identifizieren des längsten der unbeschädigten Segmente oder des Segments mit einer niedrigsten durchschnittlichen absoluten Abweichung (AAD) zwischen Atmungsspitzen oder -tälern und
das Bestimmen einer Atemfrequenz aus dem längsten unbeschädigten Segment oder dem Segment mit der niedrigsten AAD.

## Revendications

**1.** Procédé de détermination d'un rythme respiratoire à partir d'un signal représentatif d'un épisode de respiration enregistré, le procédé comprenant la fourniture d'un pointeur de début et d'un pointeur de fin destinés à indiquer une position de début et une position de fin du signal représentatif de l'épisode, et étant **caractérisé par** la mise à jour des pointeurs de début et de fin pour identifier les positions de début et de fin de deux segments non corrompus ou plus dans ledit signal, les segments non corrompus étant séparés par des segments corrompus, l'identification du plus long des segments non corrompus ou du segment ayant le plus faible écart absolu moyen, EAM, entre les pics ou les creux de respiration, et la détermination d'un rythme respiratoire à partir du segment non corrompu le plus long ou du segment ayant l'EAM le plus faible.

**2.** Procédé selon la revendication 1 comprenant en outre l'étape qui consiste pour l'utilisateur à choisir d'utiliser le segment non corrompu le plus long pour calculer le rythme respiratoire ou d'utiliser le segment ayant l'EAM le plus faible pour calculer le rythme respiratoire.

**3.** Procédé selon la revendication 1 dans lequel les segments non corrompus sont identifiés dynamiquement ou rétrospectivement.

**4.** Procédé selon l'une quelconque des revendications précédentes dans lequel l'étape d'identification des segments non corrompus comprend le fait de contrôler que le gradient et/ou l'amplitude du signal ne dépasse pas un seuil prédéterminé ou l'étape d'identification des segments non corrompus comprend l'identification de pics et/ou de creux dans le signal, l'obtention d'intervalles entre des pics adjacents et/ou des creux adjacents, le calcul de variations dans lesdits intervalles et la détermination du fait que ces variations dépassent ou non un seuil prédéterminé.

5. Procédé selon l'une quelconque des revendications précédentes dans lequel l'étape d'identification des segments non corrompus comprend le calcul d'un premier écart absolu moyen entre des pics adjacents et d'un second écart absolu moyen entre des creux adjacents, la combinaison des premier et second écarts absolus moyens pour obtenir un seuil normalisé et la détermination du fait que l'un ou l'autre des premier et second écarts absolus moyens dépasse ou non le seuil normalisé.

6. Procédé selon la revendication 5 dans lequel l'étape de combinaison des premier et second écarts absolus moyens comprend la multiplication de la somme des premier et second écarts absolus moyens par un facteur de pondération.

7. Procédé selon l'une quelconque des revendications précédentes comprenant en outre le traitement préalable des segments non corrompus avant la détermination du rythme respiratoire.

8. Procédé selon la revendication 7 dans lequel le traitement préalable comprend une ou plusieurs des étapes suivantes :

   (v) le centrage des données ;
   (vi) le réglage du gain ;
   (vii) le passage des segments dans un filtre à autoréglage ;
   (viii) le passage des segments dans un filtre passe-bas et/ou un filtre passe-haut.

9. Procédé selon l'une quelconque des revendications précédentes comprenant l'étape de vérification de la présence d'un évènement respiratoire, comprenant une ou plusieurs des étapes suivantes :

   (i) l'identification de pics et/ou de creux en dehors d'une fenêtre de marge de bruit ;
   (ii) le classement d'un pic en tant qu'évènement d'inspiration seulement s'il est précédé d'un creux d'expiration ;
   (iii) le classement d'un creux en tant qu'évènement d'expiration seulement s'il est précédé d'un pic d'inspiration ;
   (iv) l'élimination d'un évènement d'inspiration et/ou d'expiration s'il amène le rythme respiratoire à dépasser un maximum prévu ;
   (v) l'élimination d'un évènement d'inspiration et/ou d'expiration si son amplitude est inférieure à 20 %, à 30 %, à 40 % ou à 50 % de l'évènement d'inspiration et/ou d'expiration précédent.

10. Procédé selon la revendication 9 comprenant en outre la réalisation d'un contrôle de périodicité pour déterminer si la variabilité des intervalles entre deux pics adjacents et/ou deux creux adjacents dépasse un seuil prédéterminé.

11. Procédé selon l'une quelconque des revendications précédentes comprenant en outre l'étape de contrôle de validité comprenant le calcul d'un nombre de pics et/ou de creux valides détectés et l'évaluation de ce nombre pour voir s'il ou non est supérieur ou égal à un minimum prédéterminé nécessaire pour calculer le rythme respiratoire valide.

12. Procédé selon l'une quelconque des revendications précédentes comprenant en outre la vérification du fait qu'un écart absolu moyen pour un intervalle entre des pics valides adjacents ne dépasse pas un seuil prédéfini et/ou qu'un écart absolu moyen pour un intervalle entre des creux valides adjacents ne dépasse pas un seuil prédéfini.

13. Procédé selon l'une quelconque des revendications précédentes dans lequel le rythme respiratoire est calculé en tant que rythme respiratoire moyen ou médian obtenu en utilisant les évènements d'inspiration et/ou d'expiration obtenus à partir dudit segment non corrompu identifié.

14. Procédé selon l'une quelconque des revendications précédentes comprenant en outre l'émission d'un signal d'erreur lorsque le signal représentant l'épisode de respiration ne contient pas au moins deux segments de données non corrompues contiguës et/ou lorsque de quelconques valeurs calculées dépassent leurs seuils respectifs.

15. Système de surveillance respiratoire (20) permettant de surveiller la respiration afin de déterminer un rythme respiratoire à partir d'un signal représentatif d'un épisode de respiration enregistré, le système (20) comprenant :

   un capteur (22) destiné à émettre un signal représentatif d'un épisode de respiration enregistré ; et
   un processeur (24) conçu pour :

      fournir un pointeur de début et un pointeur de fin destinés à indiquer une position de début et une position de fin du signal représentatif d'un épisode de respiration enregistré ;
      le système (20) étant **caractérisé en ce que** le processeur (20) est conçu pour :

mettre à jour les pointeurs de début et de fin destinés à identifier les positions de début et de fin de deux segments non corrompus ou plus dans ledit signal, les segments non corrompus étant séparés par des segments corrompus,

identifier le plus long des segments non corrompus ou le segment ayant l'écart absolu moyen (EAM) le plus faible entre les pics ou les creux de respiration, et

déterminer un rythme respiratoire à partir du segment non corrompu le plus long ou du segment ayant l'EAM le plus faible.

16. Système selon la revendication 15 dans lequel le capteur (22) comprend un dispositif de pneumographie par impédance conçu pour émettre ledit signal.

17. Système selon la revendication 15 ou la revendication 16 conçu comme un dispositif sans fil portable et/ou un dispositif jetable fonctionnant sur pile de faible puissance.

18. Processeur (24) pour un système de surveillance respiratoire permettant de surveiller la respiration afin de déterminer un rythme respiratoire à partir d'un signal représentatif d'un épisode de respiration enregistré, ledit processeur (24) étant conçu pour :

fournir un pointeur de début et un pointeur de fin destinés à indiquer une position de début et une position de fin du signal représentatif d'un épisode de respiration enregistré, et **caractérisé par** :

la mise à jour des pointeurs de début et de fin destinés à identifier les positions de début et de fin de deux segments non corrompus ou plus dans ledit signal, les segments non corrompus étant séparés par des segments corrompus,

l'identification du plus long des segments non corrompus ou du segment ayant l'écart absolu moyen (EAM) le plus faible entre les pics ou les creux de respiration, et

la détermination d'un rythme respiratoire à partir du segment non corrompu le plus long ou du segment ayant l'EAM le plus faible.

10

```
      ┌─────────────────────────────────────────┐
12 ───┤   Obtaining a signal a signal representative of │
      │        a recorded respiration episode           │
      └─────────────────────────────────────────┘
                          │
                          ▼
      ┌─────────────────────────────────────────┐
      │ Identifying two or more uncorrupted segments within the │
14 ───┤   signal, the uncorrupted segments being separated by   │
      │              corrupted segments                 │
      └─────────────────────────────────────────┘
                          │
                          ▼
      ┌─────────────────────────────────────────┐
      │ Identifying the longest of the uncorrupted segments or the │
16 ───┤ segment with a lowest average absolute deviation (AAD) between │
      │         respiration peaks or troughs            │
      └─────────────────────────────────────────┘
                          │
                          ▼
      ┌─────────────────────────────────────────┐
      │ Determining a respiration rate from the longest uncorrupted │
18 ───┤    segment or the segment with the lowest AAD   │
      └─────────────────────────────────────────┘
```

Fig.1

20

22

26

24

30

Base
Station

Fig.2

Fig. 3

Fig.4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008275349 A